# EUROPEAN PATENT SPECIFICATION

(11) **EP 1 265 651 B1**
(45) Date of publication and mention of the grant of the patent: **03.05.2006**
(21) Application number: 01922360.1
(22) Date of filing: 13.03.2001
(51) Int. Cl.: A61L 31/08, A61L 31/18

(54) **SELECTIVE RADIOPAQUE PLATING OF A STENT**
SELEKTIVE STRAHLUNGSUNDURCHLÄSSIGE BESCHICHTUNG EINES STENTS
REVETEMENT SELECTIF D'UNE ENDOPROTHESE VASCULAIRE

(30) Priority: 20.03.2000 US 528836
(43) Date of publication of application: 18.12.2002
(73) Proprietor: Boston Scientific Limited, St. Michael, Barbados, West Indies (BB)
(72) Inventor: EIDENSCHINK, Tracee, E., J., Wayzata, MN 55391 (US); JAGGER, Karl, Alan, Deephaven, MN 55331 (US)
(74) Representative: Graalfs, Edo
(86) International application number: PCT/US2001/007955
(87) International publication number: WO 2001/070294

(56) References cited:
- EP-A- 0 836 839
- EP-A- 0 945 107
- WO-A-00/40784
- US-A- 5 607 442
- US-A- 5 725 572

## Description

### FIELD OF THE INVENTION

The present invention relates to a flexible radial expandable stent for surgical implantation in a body lumen.

Obstructive coronary artery disease is one of most serious health problems facing our society today. This disease is the result of the deposit of fatty substances on the interior surface of the walls of the arteries. The build up or lesion of such deposits results in a narrowing of the diameter of the artery which restricts the blood flow through the artery, a condition known as stenosis.

Balloon angioplasty is commonly used to open arteries to which the flow of blood has been impeded or blocked by a build-up of arterioscelerotic plaques on the interior walls of the arteries. Using this technique, a small diameter catheter that has a dilatation balloon attached to its distal end, is inserted into an artery, and guided through the vascular system to the location of the stenosis. The dilatation balloon is then inflated and flattens the plaque against the artery walls thereby opening the blocked vessel to improve the blood flow. The balloon is then deflated and removed from the patient.

A portion of the time, however, after several months or longer, these arteries undergo what is referred to as restenosis which is a reclosing of the artery at the site of the blockage. This has led to a medical procedure in which a stent is placed in the blocked artery immediately following the angioplasty procedure, and removal of the balloon. This stent is left in the artery permanently, and reduces the chance of restenosis occurring.

Stents are also commonly used in other medical procedures as well including in the repair and support of injured tissue, in urological procedures (i.e. prostate surgery for holding open tracts of the urinary system), in reproductive surgeries; and so forth.

Stents are typically short tubular structures, open at both ends, and designed for insertion into the blood vessel. Other commonly used terms included grafts, stent-grafts, and vena cava filters, which all function to serve similar purposes.

The placement and positioning of the stent is crucial during any medical procedure in which it is used. The devices are inserted through the vascular system by interventional cardiologists and radiologists who must be able to place these devices at precise locations. The position of the stent must therefore be readily ascertainable. A common means for identifying and following the precise position of a stent is to view the stent using fluoroscopy. Stents are commonly made from stainless steel, tantalum, or NITINOL (nickel-titanium alloy) with stainless steel being one of the more common materials. A major difficulty associated with stainless steel is that under certain conditions it is transparent to a fluoroscope. It is possible to position and deploy stainless steel stents by injecting radiopaque dyes into the bloodstream thereby making the vessel in which the stent is being placed visible. However, the dyes dissipate quickly allowing the vessel to be viewed for only a short period of time which requires rapid placement and deployment of the stent. Further, it is very difficult to verify that the stent has not changed location over time.

Stents may also be produced from tantalum which is, in contrast to stainless steel, readily visible using a fluoroscope and can be effectively used for identifying the location of the stent. However, tantalum fluoroscopically illuminates so brightly that it obscures the visibility of the lesion being repaired.

One way in which to solve such problems has been through the use of radiopaque coatings or markings which are commonly (conventionally) noble metals such as gold or platinum. These coatings may be affixed to the stent through electroplating.

U. S. Patent No. 5,607,442 issued March 4,1997 to Fischell et al. describes a stent plated with a high density, radiopaque metal or alloy such as gold or tantalum. The longitudinal wires of the stent are plated to a sufficient thickness to make the stent clearly radiopaque in fluoroscopy, but the generally circumferential wires are plated to a much lesser thickness so that they are not distinctly radiopaque. It is suggested at column 3, lines 47 to 49 that masking may be used to apply thicker plating to the longitudinals as compared to the rings.

U. S. Patent No. 5,725,572 issued March 10,1998 to Lam et al. describes a radiopaque stent wherein the radiopaque markers are affixed to both a distal end and a proximal end of a generally cylindrical stent by means of plating so as not to impede expansion. The stent is placed upon a mandrel, masked and plated. Shrink tubing is preferred for masking the stent. The shrink tubing may be any polyester having heat shrink properties and the ability to mask the stent during the electroplating process. See column 6, lines 2 to 5. Using this method, the curved portions of the stent as well as the ends of the mandrel are dipped in high temperature wax to prevent them from being plated. In order to plate the desired portions of the stent, the heat shrink tubing surrounding the portions of the stent to be plated is then cut away using a standard CO₂ laser or its equivalent. See column 6, lines 13 to 19.

U. S. Patent No. 5,824,045 issued October 20,1998 to Alt describes a vascular or endoluminal stent covered with a very thin, highly adherent layer of gold or other noble metal, such as platinum, or an alloy which is primarily gold or other noble metal with a considerably smaller percentage of a compatible metal which may or may not be another noble metal. Preferably, the noble metal layer is ultra-thin and covers the entire stent, including interior and exterior surfaces, and all edges bounding the internal openings in the wall. In a preferred method of the invention, a firm and lineally extensible bond is obtained between the base metal of the stent core or carrier and the noble metal of the outer layer using a conventional technique of ion deposition. The base metal is described as being typically stainless steel, and gold is suggested as the noble metal layer.

WO 00/40784 discloses a method for coating metallic articles. An oxide from the external surface of the article is removed and a coating is placed on the article. WO 00/40784 is comprised in the state of the art according Art. 54(3) (4) EPC.

EP 0 945 107 discloses a medical stent to be placed at a branch of two arteries. To assist in the placement of the stent during the stenting of a bifurcated region, radioapaque markers may be inserted in the connecting rods.

The technical problem of the invention is to provide a stent for placement in a brunch of a vessel allowing to maneuver a second stent with a precise view of the position of the opening. The problem is solved by a stent according to claim 1.

The present invention provides a tubular, flexible, expandable stent having a radiopaque material coated on the stent in such a way that it does not obstruct the view of the lesion being repaired, allows the position, diameter and length of the stent to be readily identified within a blood vessel, and does not adversely affect the expansion properties of the expandable stent.

In particular, the stent is selectively plated with a radiopaque material on at least one of its distal end or its proximal end, as well as on a portion of the body of the stent with a radiopaque material.

Preferably, the radiopaque stent of the present invention is a bifurcated stent having a radiopaque coating thereon. The portion of the body of the stent that is selectively plated with a radiopaque material marks an opening in the bifurcated stent through which a second stent may be placed. It is the surface of the stent that defines the opening that is selectively plated. The radiopaque bifurcated stents of the present invention are useful at branches in blood vessels or arteries wherein the blockage occurs at the junction of the "Y" shape.

The opening in the body of the stent, preferably in the center portion of the body, is therefore "marked" with the radiopaque coating so that the opening of the stent may be easily viewed, and the second stent subsequently positioned through the opening. It is also important that the opening of the stent does not illuminate too brightly so as to obscure the opening in the artery.

The present invention further provides an improved and convenient method of selectively plating the stents at the specified locations with a radiopaque material. The selective plating is accomplished without premasking the stent.

One of skill in the art will realize that the position of the marking may be waried, without deviating from the scope of the present invention.

Optionally, the stent may be treated in an acid bath followed by a strike layer. One or the other of these steps, or both may be performed prior to selective plating, but it is preferred that both acid etching and a strike layer are utilized.

The selective plating method is accomplished through a pen-like instrument that works through capillary action, or through the use of a syringe-like instrument that is connected to a reservoir of plating solution and is equipped with a pump for pumping the solution to the tip of the syringe, which also carries a charge.

In this manner, the stent may be plated precisely and selectively, in a sequential or coursive manner, without the need for premasking areas of the stent. A pattern may be first marked on the stent, however, prior to plating.

In contrast to the present invention, the prior art methods of plating stents required masking off either the portions that desirably remain unplated with the radiopaque marker, or masking off the areas to be plated.

For instance, some prior art methods used the masking off of certain portions of the stent that were not to be plated, with a material such as a shrink wrap, wax, tape, or some such similar material. This method further requires that these materials be removed from the stent using either chemicals, i.e. solvent, or by heat or laser techniques. Heat or lasers may be detrimental to the stent. Each of the methods can leave residual material on the stent by generation of foreign material, and laser removal of masking can cause the further complication of laser "burn through." Further, the stent is typically placed on plastic mandrels for masking, and coolant is typically run through the stent during the laser removal of the masking. Each of these added steps requires increased handling of the stent and therefore increases the possibility that the stent may be damaged during production. More steps also typically means less economic efficiency in production.

Other previously used methods of plating only portions of the stent involved plating the whole stent, masking the portions where it was desirable to retain the radiopacity on the stent, and then etching away the remaining portions to remove the radiopaque material. In any of these cases involving masking and subsequent removal of the masking material, the whole process is tedious, time consuming, and economically less efficient, particularly for very small articles such as a stent.

Furthermore, the selective plating method of the present invention has several advantages over the previously used methods in which the whole stent was coated. First, coating only portions of the stent does not increase the overall rigidity of the stent, and therefore allows the stent to remain flexible as required. Further, the coating will therefore not impair the expandability of the stent as well. Second, coating only selected portions of the stent removes the problem of the stent illuminating too brightly and obscuring the lesion that the surgeon is repairing. Third, the markers allow for the easy identification of the location of the stent, both during the surgical procedure, and in subsequent follow-up visits with the patient.

In each of the methods of the present invention, steps have been eliminated over the prior art methods, and the overall process is simplified, Further, in addition to removing steps in the plating process, less material is used in the present invention, thereby providing economic advantages as well.

The present invention therefore provides a simplified and improved method of selectively plating predetermined portions of the stent through the use of an electroplating pen, for instance. This method involves, in a sense, very precise, sequential plating of the area desired for marking termed "selective" plating herein. Previous methods, in contrast, involve methods whereby a large portion, or all of the stent, is plated at one time.

### BRIEF DESCRIPTION OF THE DRAWINGS

Fig.1 illustrates a specific embodiment of a selectively plated bifurcated radial expandable stent in an unexpanded state.
Fig. 2 is a schematic diagram illustrating a series of process steps that may be involved in electroplating a stent using the method of the present invention.
Fig. 3 is a representation of a WIER Spillway Dip.

### DETAILED DESCRIPTIONS OF THE PREFERRED EMBODIMENTS

While this invention may be embodied in many different forms, there are described in detail herein specific preferred embodiments of the invention. This description is an exemplification of the principles of the invention and is not intended to limit the invention to the particular embodiments illustrated.

Generally, the stents useful herein include tubular, flexible, expandable vascular or endoluminal stents adapted for deployment in a vessel or tract of a patient to maintain an open lumen. The stents are typically radially expandable stents formed from either a hollow tube, or a sheet formed from a biocompatible metal such as stainless steel, nitinol (nickel-titanium alloy), and so forth. The stent has a multitude of openings in the stent wall, and is open at both the proximal and the distal end. The stents are typically formed from a single member, although this is not always the case as with wire stents.

The stents are fabricated having a predetermined inner diameter in a production state and are adapted for expansion to a larger diameter upon deployment in a vessel or tract.

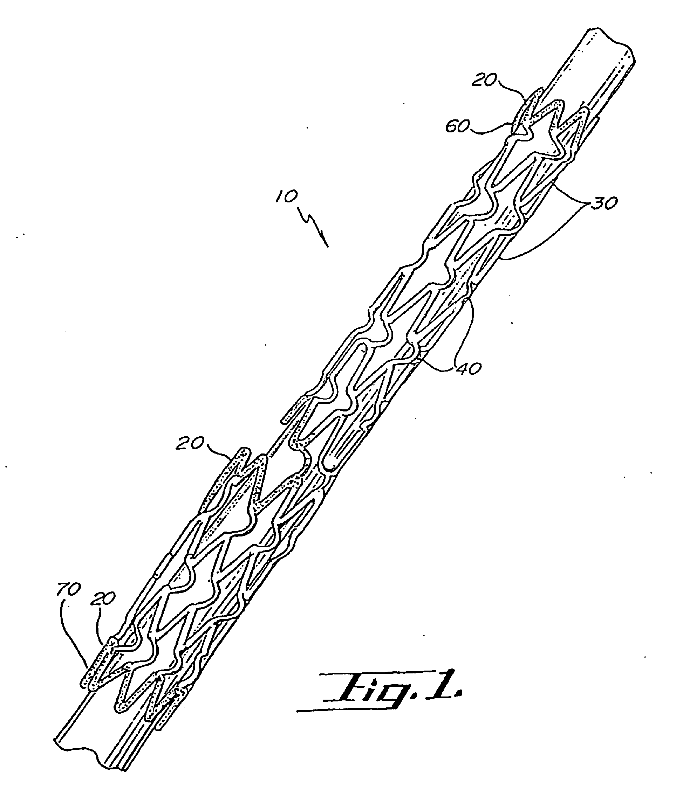

Fig. 1 illustrates generally at 10 a specific preferred flexible radially expandable stent in its unexpanded state. The stent is typically formed of a plurality of adjacent band-like or adjacent zig-zagging or undulating elements 30 which form a serpentine, zig-zagging or undulating flexible pattern. Hereinafter referred to as a zig-zagging pattern. The zig-zagging band-like elements are interconnected with straight or curvilinear interconnecting U-shaped members 40 extending between adjacent bands 30. The bands are arranged in a plurality of interconnected flexible rows disposed along the longitudinal axis of the stent. The distal end of the stent has a distal row disposed thereon and the proximal end of the stent has a proximal row disposed thereon.

The zig-zagging elements 30 have a selected wavelength or wavelengths, and are aligned along a common longitudinal axis to define a generally tubular stent body. The zig-zagging elements 30 may take a generally longitudinal direction along the stent body or may be slanted relative to the longitudinal axis of the stent. Adjacent zig-zagging elements 30 may be in phase or out of phase with each other.

The stents further comprise a plurality of interconnecting U-shaped elements 40 having first and second ends. The first and second ends extend from adjacent zig-zagging elements 30 and are displaced from one another in a longitudinal direction and optionally in a radial direction along the stent. The interconnecting elements 40 may be angularly disposed with respect to the longitudinal axis of the stent. The interconnecting elements 40 may be straight of curvilinear.

Optionally, upon expansion of the stent, at least some of the zig-zagging elements 30 may be displaced relative to each other about the periphery of the stent to accommodate longitudinal flexing of the stent within the zig-zagging elements 30 and without interference between adjacent elements.

The stent further is bifurcated, being designed for placement at a branch in an artery or vessel of the patient. Sometimes the build-up of fatty substances or lesions that restrict the blood flow of an artery may form at the intersection between two arteries, that is, where the section where the two arteries form a generally "Y" configuration (e.g. bifurcate, trifurcate, and so on).

In this particular case, the stent has an opening 50 through which a second stent may be maneuvered and subsequently positioned at the branch. The two stents will therefore substantially form the "Y" shape at the obstructed intersection so that one stent may be placed in the first branch and the second stent may be placed in the second branch. The second stent may be placed in the vessel or artery during a subsequent procedure, or during the same procedure but following the placement and expansion of the first stent.

The stent therefore advantageously has a radiopaque coating 20, located at the distal end 60, at the proximal end 70 and around the opening 50. The distal end 60 has a smaller diameter than the proximal end 70, the distal end being inserted into the patient first. The stent can therefore not only be located, but the orientation of the stent may also be determined accurately using fluoroscopy. This is important for proper placement of the second stent through the opening of the first stent and into the branch of the vessel.

Stents, subsequent to placement in an artery, may reposition themselves. It is therefore necessary for the surgeon to be able to accurately judge if the opening 50 through which the second stent is to be maneuvered is aligned properly at the branch in the artery or vessel.

The present invention is most particularly useful for bifurcated stents such as these, wherein the stent has an opening through which the second stent may be maneuvered. The radiopaque marking of this opening is an extremely desirable feature for the bifurcated stent to have.

Radial expandable stents are described generally in Application Serial Nos. O8/511,076; 09/111,531; and 09/197,276 all now pending, the entire contents of which are herein incorporated by reference.

Preferably, both the proximal 70 and the distal 60 ends of the stent are coated with a radiopaque material. Alternatively, however, at least one of the proximal end 70 and the distal end 60 are selectively plated with a radiopaque material. As discussed above, a portion of the center of the stent, is also be plated with a radiopaque material.

Other radial expandable stents are described generally in U.S. Patent Nos. 5,807,404 issued September 15, 1998 to Richter, and 5,836,964 issued November 17, 1998 and 5,922,005 issued July 13, 1999 both to Richter et al., the entire contents of which are herein incorporated by reference.

A particularly preferred stent for use in the present invention is a bifurcated stent but using the general structures of such stents as desribed above. One of skill in the art will realize that these are only suggested types of stents, and are in no way intended to limit the scope of the present invention.

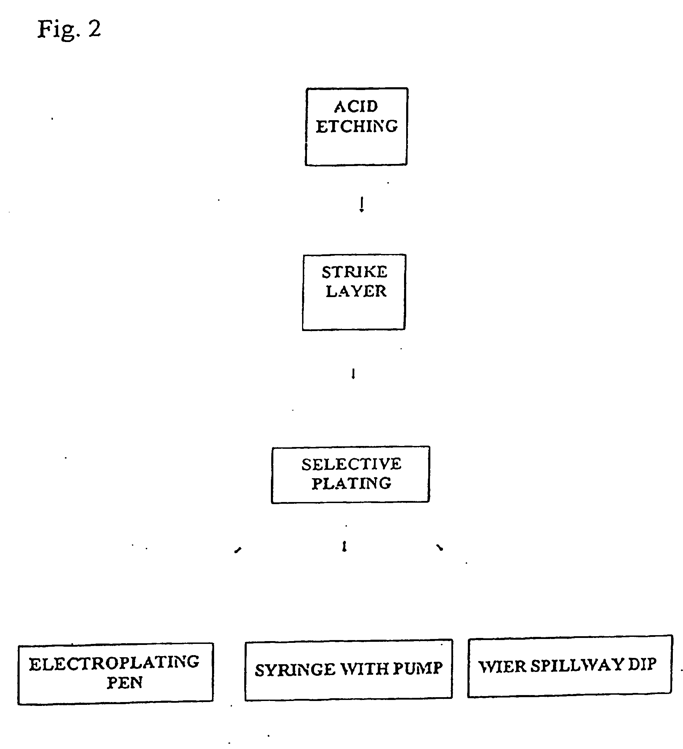

Fig. 2 is a general illustration of the steps that may be involved in the present invention. The first and second steps are optional. The third step is the selective plating step and may be accomplished using a number of different techniques including an electroplating pen that works by capillary action, a syringe connected to a pump, and a controlled dip process.

During the first step, the stent may be placed in an acid bath prior to electroplating to remove oxides from the surface of the metal. This may be referred to as acid etching, and as stated above, is optional. Using this technique, oxides are removed from the surface of the metal stent. The use of this technique is known to one of skill in the art.

During the second step, a "strike" layer is optionally applied. The strike layer is a very thin electrochemically deposited layer that prevents reformation of oxides on the surface of the metal thereby improving the adhesion of the subsequent coating. This step must immediately follow the acid bath so that the oxides do not get an opportunity to reform on the surface of the metal.

The strike layer may be any metal typically used for a "strike" including the noble metals, nickel and so forth. It is preferable to the present invention to use a gold "strike." Gold is known to produce less thrombus, tissue irritation and/or allergic reaction, and so forth than other metals. It is preferable to the present invention to apply a "strike" layer prior to selectively plating the stent.

During the strike, the stent is plated with a solution of a metal salt, preferably gold wherein a thin layer of gold, i.e. the "strike", is immediately deposited on the surface of the stent. This layer is controlled by the concentration of the solution, the time of exposure, and the amperage of the current, to a thickness of preferably about 5 x 10⁻⁶ cm to about 1.5 x 10⁻⁵ cm (about 0.5 *µ*m to about 1.5*µ*m; about 500 to about 1500 Å). A typical strike layer is approximately 1.3 x 10⁻⁵ cm (about 1.3 *µ*m or 1270 Å). This thin layer has no affect on the rigidity or expansion properties of the stent thereby allowing the entire surface to be coated with a simple immersion step. The "strike" layer is added to improve the adhesion of the subsequently applied radiopaque coating to the stent.

The very thin "strike" layer allows the entire stent to be dipped in a plating solution of the metal because the thin coating will not have an adverse affect on the properties, such as the flexibility or increased radiopacity, of the stent.

The radiopaque material is then electroplated selectively over the "strike" layer using the selective plating methods as described above. The radiopaque layer will not be electroplated over the whole strike layer, but only over the selected, predetermined areas.

The method of selectively plating the stents of the present invention involves the use of a pen-like instrument, or a syringe-like instrument, for example, and may be accomplished either by hand, or may be automated. For instance, robotics may be used to selectively apply the radiopaque coating.

The method of the present invention utilizes electrically charged pens of which can be used to "draw" or "write" on the stent where the radiopaque material is desirably plated. The electoplating pens contain radiopaque material such as gold. The surface of the stent is oppositely charged from the plating metal, thereby leading to an attractive force between the surface of the stent and the radiopaque material. Electroplating pens are available from a variety of sources one such source being Hunter Products, Inc. in Bridgewater, NJ.

Alternatively, the electroplating pen may be modified to a hand applied system wherein a positive flush of gold plating solution is supplied to a disposable tip applicator. Whereas pens rely on capillary action, a modified pen may be similar to a syringe. The plating solution is pumped from a reservoir that is connected to the syringe, and out through a charged nozzle. The amount or thickness of the deposition can be adjusted by flow control, i.e. the amount of solution being pumped to the tip of the pen can be adjusted.

In the above methods wherein a pen-like instrument or syringe-like instrument is used, the plating is accomplished in a sequential or coursive manner, much like writing. The whole stent is not dipped in a solution at once, but rather the area which is being plated is quite controlled.

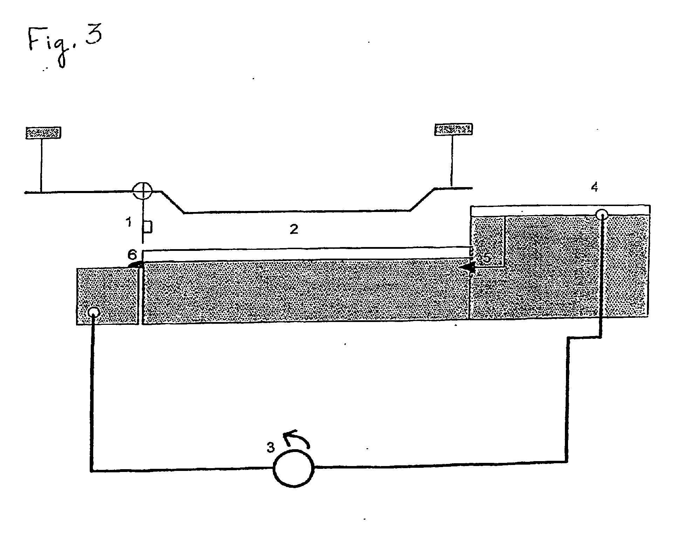

Alternatively, a reservoir of plating solution may be maintained at a precise level of solution. The stent is lowered into or moved through the reservoir having the controlled and precise level of electrochemical solution. The stent is placed into the reservoir to a specified depth thereby plating only a selected portion of the stent. The stent will therefore be immersed in the solution only to a certain depth. A WIER Spillway Dip is an example of such a method. This method is useful for plating the proximal and distal ends. Fig. 3 is a representation of a spillway dip. The stent is held at the stent electrical contact and holding fixture 1 and is conveyed through conveyer immersion area 2. The depth of the immersion area 2 is controlled by the WEIR Spillway immersion level control 6. A circulating pump 3 keeps the solution circulating through the reservoir 4 and the immersion area 2 through a filtration and positive pressure flow device 5.

In each of the above methods, as well as variations of such methods, the need for premasking of the stent prior to plating has been eliminated.

The pattern of plating will be selected prior to application of the radiopaque layer. The radiopaque coating is preferably a noble metal and is even more preferably gold, and may also be a mixture or alloy of metals as well.

The thickness of the coating may be varied, but is preferably between about 1 µm to about 20 µm (about 3.9 x 10⁻⁵ inches to about 7.9 x 10⁻⁴ inches), preferably from about 2 µm to about 12*µ*m (about 7.9 x 10⁻⁵ inches to about 4.7 x 10⁻⁴ inches). The desired thickness may be achieved, for instance, through one or more electrochemical depositions.

Using this method of plating only portions of the stent eliminates wash out or haloing. These phenomena can occur where the whole stent is either itself formed of a radiopaque material, such as tantalum, or the entire stent is coated with a relatively thick layer of a radiopaque material. This makes the stent difficult to view under fluoroscopy because it illuminates too brightly, making the lines of the stent indistinct, and not clearly discernible. Furthermore, it makes it difficult to accurately deternnine the location and orientation of the stent. Also, if the stent is very luminous, the haloing can obscure visibility of the blood vessel lesion, and can actually mask it, making it difficult to repair the vessel.

Further to the present invention, selective plating allows the expansion characteristics of the stent to be controlled. For instance, application of the coating layer in certain areas can increase the rigidity of the stent in those areas. This can result in either a "watermelon seed" effect wherein the stent shoots from the desired location of stent placement, or if the middle deploys first, the stent may lodge in the vessel. It has been noted that the coating can increase the rigidity of the stent in those areas where it is plated, and can result in the balloon opening the center or body of the stent first if it is also not plated. The thicker the coating, the more likely that the stent may deploy first at those portions where the coating is not as thick. With the method of the present invention, the stent can be balanced easily by selectively plating certain areas, thereby preventing undesirable movement of the stent. For instance, if a portion of the middle of the stent is plated, as well as the distal and proximal ends, the center will have less of a tendency to open first. Furthermore, the thickness to which the areas are plated can be easily controlled. For instance, certain portions of the stent may be plated with a thicker coating, causing the stent to illuminate more brightly in certain areas. This would allow not only the location of the stent to be easily determined, but the orientation of the stent as well.

The current invention allows the use of a base stainless steel stent which maintains good flexibility. The application of the radiopaque material to only the ends or specified subsections does not increase stent rigidity.

In addition to being directed to the embodiments described above and claimed below, the present invention is further directed to embodiments having different combinations of the features described above and claimed below. As such, the invention is also directed to other embodiments having any other possible combination of the dependent features claimed below.

The above figures and disclosure are intended to be illustrative and not exhaustive, and will suggest many variations and alternatives to one of ordinary skill in this art.

## Claims

1. A radiopaque stent (10) for placement in a branch of a vessel, said stent having a proximal end, a distal end and a body, said body having an opening (50) through which a second stent may be maneuvered, said opening having a surface defining said opening, **characterized in that** said surface of said opening, and at least one of said proximal end and said distal end are selectively plated with a radiopaque material.

2. The stent of Claim 1 wherein said radiopaque material is selected from the group consisting of gold, platinum, silver, tantalum, iridium, rhodium, and mixtures and alloys thereof.

3. The stent of Claim 2 wherein said radiopaque material is gold.

4. The stent of Claim 1 wherein said radiopaque material is selectively plated to a thickness of about 2 µm to about 20 µm.

5. The stent of Claim 1 wherein a strike layer is first applied to said stent.

6. The stent of Claim 5 wherein said strike layer is gold.

7. The stent of Claim 5 wherein said strike layer has a coating thickness of about 0.5 µm to about 1.5 µm.

8. The stent of Claim 1 wherein said selective plated stent is not premasked.

## Patentansprüche

1. Ein röntgendichter Stent (10) zur Platzierung an der Verzweigung eines Körpergefäßes, wobei der besagte Stent ein nahes Ende, ein fernes Ende und einen Körper besitzt, der besagte Körper eine Öffnung (50) besitzt, durch die ein zweiter Stent manövriert werden kann, die besagte Öffnung eine Oberfläche besitzt, die die besagte Öffnung begrenzt, und **dadurch gekennzeichnet ist, dass** die besagte Oberfläche der besagten Öffnung und zumindest eines der besagten nahen und fernen Enden selektiv mit einem röntgendichten Material beschichtet sind.

2. Der Stent gemäß Anspruch 1, wobei das besagte röntgendichte Material aus einer Gruppe ausgewählt ist, die aus Gold, Platin, Silber, Tantal, Iridium, Rhodium und Gemischen und Legierungen hiervon besteht.

3. Der Stent gemäß Anspruch 2, wobei das besagte Material Gold ist.

4. Der Stent gemäß Anspruch 1, wobei das besagte röntgendichte Material wahlweise bis zu einer Dicke von etwa 2*µ*m bis zu etwa 20*µ*m aufgetragen ist.

5. Der Stent gemäß Anspruch 1, wobei zuerst eine Überzugschicht auf den besagten Stent aufgetragen ist.

6. Der Stent gemäß Anspruch 5, wobei die besagte Überzugschicht aus Gold besteht.

7. Der Stent gemäß Anspruch 5, wobei die besagte Überzugschicht eine Beschichtungsdicke von etwa 0,5 µm bis zu etwa 1,5 µm aufweist.

8. Der Stent gemäß Anspruch 1, wobei der besagte selektiv beschichtete Stent vor der Beschichtung nicht abgedeckt wird.

## Revendications

1. Endoprothèse vasculaire radio-opaque (10) destinée à être placée dans une branche d'un vaisseau, ladite endoprothèse vasculaire ayant une extrémité proximale, une extrémité distale et un corps, ledit corps ayant une ouverture (50) à travers laquelle une seconde endoprothèse vasculaire peut être manoeuvrée, ladite ouverture ayant une surface définissant ladite ouverture, **caractérisée en ce que** ladite surface de ladite ouverture et au moins l'une de ladite extrémité proximale et de ladite extrémité distale sont plaquées de manière sélective avec un matériau radio-opaque.

2. Endoprothèse vasculaire selon la revendication 1, dans laquelle ledit matériau radio-opaque est sélectionné dans le groupe constitué par l'or, le platine, l'argent, le tantale, l'iridium, le rhodium et des mélanges ou alliages de ceux-ci.

3. Endoprothèse vasculaire selon la revendication 2, dans laquelle ledit matériau radio-opaque est de l'or.

4. Endoprothèse vasculaire selon la revendication 1, dans laquelle ledit matériau radio-opaque est plaqué de manière sélective à une épaisseur d'environ 2 µm à environ 20 µm.

5. Endoprothèse vasculaire selon la revendication 1, dans laquelle une couche d'amorce est tout d'abord appliquée à ladite endoprothèse vasculaire.

6. Endoprothèse vasculaire selon la revendication 5, dans laquelle ladite couche d'amorce est en or.

7. Endoprothèse vasculaire selon la revendication 5, dans laquelle ladite couche d'amorce a une épaisseur de revêtement d'environ 0,5 µm à environ 1,5 µm.

8. Endoprothèse vasculaire selon la revendication 1, dans laquelle ladite endoprothèse vasculaire plaquée de manière sélective n'est pas prémasquée.
